(19) Europäisches Patentamt
European Patent Office
Office européen des brevets

(11) Veröffentlichungsnummer: **0 104 296**
**B1**

(12) **EUROPÄISCHE PATENTSCHRIFT**

(45) Veröffentlichungstag der Patentschrift: **18.12.85**

(51) Int. Cl.⁴: **C 07 C 47/548,** C 07 C 45/72

(21) Anmeldenummer: **82810395.2**

(22) Anmeldetag: **23.09.82**

(54) **Verfahren zur Herstellung von Stilben-4,4'-dialdehyd.**

(43) Veröffentlichungstag der Anmeldung:
**04.04.84 Patentblatt 84/14**

(45) Bekanntmachung des Hinweises auf die Patenterteilung:
**18.12.85 Patentblatt 85/51**

(84) Benannte Vertragsstaaten:
**CH DE FR GB IT LI**

(56) Entgegenhaltungen:
**JOURNAL OF ORGANIC CHEMISTRY, Band 43, Nr. 12 9. Juni 1978 J.E. PLEVYAK et al. "Palladium-catalyzed Arylation of Ethylene" Seiten 2454-2456**

(73) Patentinhaber: **CIBA-GEIGY AG, Klybeckstrasse 141, CH-4002 Basel (CH)**

(72) Erfinder: **Reinehr, Dieter, Dr., Wolfsheule 10, D-7842 Kandern (DE)**
Erfinder: **Spencer, Alwyn, Dr., Schützengraben 15, CH-4051 Basel (CH)**

**EP 0 104 296 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Die Erfindung betrifft ein neues Verfahren zur Herstellung von Stilben-4,4'-dialdehyd.

Vinylisch oder allylisch substituierte organische Verbindungen, u.a. Styrole und/oder Stilbene, können durch katalytische Umsetzung von entsprechenden Halogeniden mit Olefinen, z.B. Acrylsäuremethylester oder Aethylen, in Gegenwart von tertiären Aminen hergestellt werden. Als Katalysatoren werden bevorzugt Gemische von Palladiumcetat und Triphenylphosphin oder Tri-o-tolyphosphin verwendet. Die Umsetzung kann mit oder ohne Zusatz von organischen Lösungsmitteln, wie Methanol, Acetonitril, N,N-Dimethylformamid, N,N-Dimethylacetamid oder überschüssigem Olefin, vorgenommen werden. Bei der Umsetzung von Halogenbenzolen mit Aethylen unter Druck entstehen je nach Reaktonsbedingungen und/oder Ausgangs-Halogenbenzolen Styrole und/oder Stilbene [vgl. z.B. U.S. Patentschrift 3.922.299 und J.Org. Chem., 43, 2454 und 43, 2941 (1978)]. Gemäss Bull. Chem., Soc. Japan, 46, 1505 (1973) lassen sich verschiedene Olefine, u.a. Aethylen oder Propylen, in Gegenwart von Palladiumschwarz oder $PdCl_2$ und einem Überschuss an Kaliumacetat als Säureakzeptor mit methanolischen Lösungen von Halogenbenzolen, besonders Jodbenzolen, arylieren. Bei diesen vorbekannten Verfahren wird die Palladiumverbindung in einer Menge von mindestens 1 Mol%, bezogen auf das Halogenbenzol, eingesetzt.

Es wurde ein Verfahren zur Herstellung von Stilben-4,4'-dialdehyd durch Umsetzung von Aethylen mit 4-Brombenzaldehyd in Gegenwart einer Base und einer Palladiumverbindung als Katalysator gefunden, welches dadurch gekennzeichnet ist, dass man die Umsetzung bei einem Aethylen-Partialdruck von 0,01 bis 1 bar vornimmt.

Nach dem erfindungsgemässen Verfahren lässt sich das Stilben-4,4'-dialdehyd auf einfache, wirtschaftliche Weise unter milden Reaktionsbedingungen und unter Verwendung des leicht zugänglichen 4-Brombenzaldehyd herstellen. Dabei wurde unter sehr geringen Aethylen-Partialdrukken nach überraschend kurzen Reaktionszeiten eine unerwartet hohe Ausbeute erzielt.

Vorzugsweise wird die Umsetzung bei einem Aethylen-Partialdruck von 0,1 bis 1,0 bar vorgenommen. Die definitionsgemässen Aethylen-Partialdrucke lassen sich beispielsweise durch Einleiten von Aethylen in die Reaktionslösung unter Normaldruck oder durch Vorlegen eines Inertgases in geschlossenen Reaktoren erzielen.

Die Katalysator-Typen sowie der 4-Brombenzaldehyd sind bekannt. Nicht bekannte Katalysatoren können nach an sich bekannten Methoden hergestellt werden. Das Aethylen und der 4-Brombenzaldehyd werden in mindestens stöchiometrischer Menge eingesetzt.

Als Base können im erfindungsgemässen Verfahren Verbindungen der Formel

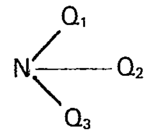 (I) oder 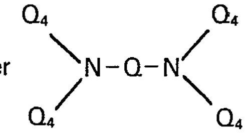 (II)

verwendet werden, worin Q geradkettiges oder verzweigtes $C_{2-6}$-Alkylen, $Q_1$ $C_{1-12}$-Alkyl, Cyclopentyl, Cyclohexyl, Phenyl oder Benzyl, das auch substituiert sein kann, beispielsweise durch ein Halogenatom, wie Chlor oder Brom, oder eine $C_{1-4}$-und besonders $C_{1-2}$-Alkyl- oder Alkoxygruppe, $Q_2$ und $Q_3$ gleiches oder verschiedenes $C_{1-12}$-Alkyl und $Q_4$ Methyl oder Aethyl bedeuten. Alkylgruppen $Q_1$ bis $Q_4$ können geradkettig oder verzweigt sein. Stellen $Q_1$ bis $Q_3$ Alkylgruppen dar, so weisen diese mit Vorteil zusammen mindestens 9 C-Atome auf. Bevorzugt werden Verbindungen der Formel (I) eingesetzt.

Beispiele von Verbindungen der Formeln I und II sind: Triäthylamin, Tri-n-butylamin, Tri-(2-äthylhexylamin), Tri-n-octylamin und Tri-n-dodecylamin; N-Benzyldialkylamine, wie N-Benzyldimethylamin, N-Benzyldiäthylamin, N-(4-Chlorbenzyl)-dimethylamin und N-(3-Methyl- oder 3-Methoxybenzyl)-dimethylamin; N,N,N',N'-Tetramethyl- und N,N,N',N'-Tetraäthyl-äthylendiamin, N,N,N',N'-Tetramethyl-1,3-diaminopropan und N,N,N',N'-Tetramethyl-1,6-diaminohexan. Bevorzugt verwendet man Tri-n-butylamin.

Die Basen werden in mindestens stöchiometrischer Menge eingesetzt. Vorzugsweise verwendet man einen Überschuss an Base, z.B. bis ca. 5 Mol Base, insbesondere 1,25 Mol Base, bezogen auf das 4-Brombenzaldehyd.

Die Reaktionstemperaturen für die erfindungsgemässe Umsetzung liegen zweckmässig zwischen 30 und 200° C, vorzugsweise zwischen 80 und 150° C. Bevorzugt wird die Reaktion in einem gegenüber den Reaktionspartnern inerten organischen Lösungsmittel durchgeführt. Geeignete inerte organische Lösungsmittel sind cyclische, oder N,N-disubstituierte Amide vor allem Verbindungen der Formel III

$$Q_5Q_6NCOQ_7 \qquad (III)$$

worin $Q_5$ und $Q_6$ unabhängig voneinander geradkettiges oder verzweigtes $C_{1-8}$-Alkyl, $C_{5-8}$-Cycloalkyl oder Benzyl oder zusammen $-(CH_2)_3-$, $-(CH_2)_4-$ oder $-(CH_2)_2-O-(CH_2)_2-$ darstellen und $Q_7$ Wasserstoff, geradkettiges oder verzweigtes $C_{1-8}$-Alkyl oder zusammen mit $Q_5$ $-(CH_2)_q-$ mit q = 3, 4 oder 5 bedeutet.

Alkylgruppen $Q_5$ und $Q_6$ weisen bevorzugt 1-5 und insbesondere 1-3 C-Atome auf. Stellen $Q_5$ und/oder $Q_6$ Cycloalkylgruppen dar, so handelt es sich insbesondere um Cyclopentyl oder Cyclohexyl. Alkylgruppen $Q_3$ weisen bevorzugt 1 oder 2 C-Atome auf. Als Beispiele von Verbindungen der Formel III seien genannt: N,N-Dimethylformamid, N,N-Diäthylformamid, N,N-Di-n-butylformamid, N,N-Diisopentylformamid, N,N-Dimethylacetamid, N,N-Dimethylpropionamid, N-Methyl-N-benzylformamid, N-Aethyl-N-cyclohexylformamid, N-Formylpiperidin, N-Formylpyrrolidin, N-Acetylmorpholin, N-Methylpyrrolidon, N-Aethylpyrrolidon und N-Methylpiperidon. Bevorzugt verwendet man als Lösungsmittel N,N-Dimethylformamid, N,N-Diäthylformamid, N,N-Dimethylacetamid oder N-Methylpyrrolidon. Ganz besonders bevorzugt ist, N,N-Dimethylformamid. Als Lösungsmittel kann man auch N,N,N',N'-Tetramethylharnstoff verwenden.

Als Katalysatoren können z.B. Palladiumkomplexe der in der U.S. Patentschrift 3.922.299 beschriebenen Art, vor allem Palladium(II)-Komplexe, wie z.B. von $PdCl_2$, $PdBr_2$, $Pd(CN)_2$, $Pd(NO_3)_2$, $Pd(CH_3COCHCOCH_3)_2$, $Pd(OOC-C_{1-12}-Alkyl)_2$, besonders Palladiumacetat, oder auch Palladium(O)-Komplexe, wie z.B. Komplexe von Bis-(Dibenzylidenaceton)palladium(O) und Bis-(Phenylisonitril)palladium(O), mit 3-wertigen Phosphor- oder Arsen-Verbindungen, wie Trialkyl-, Triaryl-, Trialkoxy- und Triphenoxyphosphinen oder -arsinen oder gemischt substituierten dreiwertigen Phosphor- oder Arsenverbindungen, verwendet werden. Als Beispiele solcher Phosphor- oder Arsenverbindungen seien genannt: Triphenylarsin, Diphenylmethylphosphin, Diphenylmethoxyphosphin, Trimethylphosphin, Triäthylphosphin, Tri-n-butylphosphin, Triphenylphosphin, Phenyl-di-n-butoxyphosphin, Triphenylphosphit und besonders Tri-o-tolylphosphin. Die genannten Komplexe können als solche eingesetzt oder in situ, d.h. im Reaktionsmedium, gebildet werden.

Der Phosphor- bzw. Arsenligand wird zweckmässig in 2-10-fachem molarem Überschuss, bezogen auf das Palladium, verwendet. Bevorzugt verwendet man als Katalysatoren Gemische aus $PdCl_2$ oder Palladiumacetat und Tri-n-butylphosphin, Triphenylphosphin, Tri-o-tolylphosphin oder Triphenylphosphit. Besonders bevorzugt sind Gemische aus Palladiumacetat und Triphenylphosphin oder Tri-o-tolylphosphin.

Der erfindungsgemäss herstellbare Stilben-4,4'-dialdehyd kann zur Herstellung bekannter optischer Aufheller eingesetzt werden. Derartige optische Aufheller sind z.B. in der U.S. Patentschrift 4.108.887 beschrieben. Die erfindungsgemäss hergestellten Verbindungen können ferner auf an sich bekannte Weise, gegebenenfalls unter Einführung von geeigneten funktionellen Gruppen, wie Aminogruppen, und/oder durch Sulfonierung der aromatischen Reste in Farbstoffe oder optische Aufheller übergeführt werden [vgl. z.B. Encyclopedia of Chemical Technology, 2. Auflage Bd. 19, S. 1 bis 16]. Stilben und Stilbenderivate finden auch Anwendung als Scintillatoren, Klebstoffadditive, Insektizide oder Lichtstabilisatoren; vgl. z.B. Chemical Abstracts 78, 39352; 84, 137386 und 85, 22416.

Die folgenden Beispiele veranschaulichen die Erfindung, ohne sie darauf zu begrenzen. Umsatzzahl = $\frac{\text{Mol Endprodukt}}{\text{Mol Pd-Verbindung}}$.

*Beispiel 1*

Unter Argon wird eine Stammlösung von 0,02244 g (0,0001 Mol) Palladiumacetat und 0,1216 g (0,0004 Mol) Tri-(o-tolyl)-phosphin in 20 ml Dimethylformamid hergestellt. In einem Rückflussapparat werden unter Aethylen 9,5 ml Dimethylformamid vorgelegt und 4,63 g (25 mMol) 4-Brombenzaldehyd, 6,56 mol (22,5 mMol) Tri-n-butylamin und 0,5 ml Stammlösung zugegeben. Das Gemisch wird 8 Stunden bei 130° C, unter gleichzeitigem Durchleiten von Aethylen gerührt. Anschliessend wird das Reaktionsgemisch mit 20 ml Wasser versetzt und der ausgeschiedene Stilben-4,4'-dialdehyd abfiltriert. Man erhält 2,25 g (9,5 mMol) Stilben-4,4'-dialdehyd, entsprechend einer Ausbeute von 76% d.Th., als gelbe Kristalle vom Fp. 170-172° C. (Pd-Gehalt 0,01 Mol%, bezogen auf 4-Brombenzaldehyd; Umsatzzahl 7600 bezogen auf 4-Brombenzaldehyd).

*Beispiel 2*

Es wird wie in Beispiel 1 beschrieben vorgegangen, jedoch unter Verwendung von 4,14 ml (27,5 mMol) N-Benzyldimethylamin anstelle von Tri-n-butylamin. Man erhält 1,77 g (7,5 mMol) Stilben-4,4'-dialdehyd, entsprechend einer Ausbeute von 60% d.Th. (Pd-Gehalt 0,01 Mol%; Umsatzzahl 6000).

*Beispiel 3*

Es wird wie in Beispiel 1 beschrieben, vorgegangen, jedoch unter Verwendung von N,N-Dimethylacetamid anstelle von Dimethylformamid. Man erhält 2,37 g (10,0 mMol) Stilben-4,4'-dialdehyd, entsprechend einer Ausbeute von 80% d.Th. (Pd-Gehalt 0,01 Mol%; Umsatzzahl 8000).

*Beispiel 4*

Es wird wie in Beispiel 1 beschrieben, vorgegangen, jedoch unter Verwendung von N-Methylpyrrolidon anstelle von Dimethylformamid. Man erhält 2,22 g (9,4 mMol) Stilben-4,4'-dialdehyd, entsprechend einer Ausbeute von 75% d.Th. (Pd-Gehalt 0,01 Mol%; Umsatzzahl 7500).

*Beispiel 5*

Es wird wie in Beispiel 1 beschrieben, vorgegangen, jedoch unter Verwendung von N,N,N'N'-Tetramethylharnstoff anstelle von Dimethylformamid. Man erhält 2,05 g (8,7 mMol) Stilben-4,4'-dialdehyd, entsprechend einer Ausbeute von 69% d.Th. (Pd-Gehalt 0,01 Mol%; Umsatzzahl 6900).

*Beispiel 6*

Es wird wie in Beispiel 1 beschrieben, vorgegangen, jedoch unter Verwendung von 0,2 ml Stammlösung und 9,8 ml Dimethylformamid. Nach einer Reaktionszeit von 16 Stunden erhält man 2,33 g (9,9 mMol) Stilben-4,4'-dialdehyd, entsprechend einer Ausbeute von 79% d.Th. (Pd-Gehalt 0,004 Mol%; Umsatzzahl 19750).

*Beispiel 7*

Es wird wie in Beispiel 1 beschrieben, vorgegangen, jedoch unter Verwendung von 2,5 ml

Stammlösung und 7,5 ml Dimethylformamid. Man erhält 2,22 g (9,4 mMol) Stilben-4,4'-dialdehyd, entsprechend einer Ausbeute von 75% d.Th. (Pd-Gehalt 0,05 Mol%; Umsatzzahl 1500).

## Patentansprüche

1. Verfahren zur Herstellung von Stilben-4,4'-dialdehyd durch Umsetzung von Aethylen mit 4-Brombenzaldehyd in Gegenwart einer Base und einer Palladiumverbindung als Katalysator, dadurch gekennzeichnet, dass man die Umsetzung bei einem Aethylen-Partialdruck von 0,01 bis 1 bar durchführt.

2. Verfahren nach Anspruch 1, dadurch gekennzeichnet, das man die Umsetzung in Gegenwart eines Gemisches aus Palladiumacetat und Tri-o-tolylphosphin als Katalysator durchführt.

3. Verfahren nach Anspruch 2, dadurch gekennzeichnet, dass man die Umsetzung bei einem Aethylen-Partialdruck zwischen 0,1 und 1,0 bar durchführt.

4. Verfahren nach Anspruch 3, dadurch gekennzeichnet, dass man den Katalysator in einer Menge von 0,01 bis 2 Mol%, bezogen auf den 4-Brombenzaldehyd, verwendet.

5. Verfahren nach den Ansprüchen 1 und 4, dadurch gekennzeichnet, dass man die Umsetzung bei einer Temperatur zwischen 30 und 200° C, vorzugsweise zwischen 80-150° C und in Gegenwart eines gegenüber den Reaktionspartnern inerten organischen Lösungsmittels vornimmt.

6. Verfahren nach Anspruch 5, dadurch gekennzeichnet, dass man als Lösungsmittel cyclische oder N,N-disubstituierte Amide verwendet.

7. Verfahren nach den Ansprüchen 1 und 6, dadurch gekennzeichnet, dass man die Umsetzung in Gegenwart einer Base durchführt.

8. Verfahren nach Anspruch 7, dadurch gekennzeichnet, dass man als Base eine Verbindung der Formeln

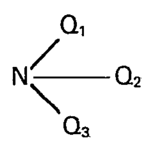 (I) oder 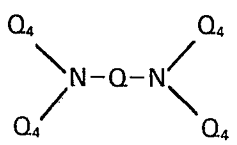 (II)

verwendet, worin Q geradkettiges oder verzweigtes $C_{2-6}$-Alkylen, $Q_1$ $C_{1-12}$-Alkyl, Cyclopentyl, Cyclohexyl, Phenyl oder Benzyl, das auch substituiert sein kann, beispielsweise durch ein Halogenatom, wie Chlor oder Brom oder eine $C_{1-4}$- und besonders $C_{1-2}$-Alkyl- oder Alkoxygruppe, und $Q_2$ und $Q_3$ gleiches oder verschiedenes $C_{1-12}$-Alkyl und $Q_4$ Methyl oder Aethyl bedeuten.

9. Verfahren nach Anspruch 8, dadurch gekennzeichnet, dass man als Base Tri-n-butylamin verwendet.

10. Verfahren nach Anspruch 6, dadurch gekennzeichnet, dass man als Lösungsmittel Verbindungen der Formel

$$Q_5Q_6NCOQ_7$$

verwendet, worin $Q_5$ und $Q_6$ unabhängig voneinander geradkettiges oder verzweigtes $C_{1-8}$-Alkyl, $C_{5-8}$-Cycloalkyl oder Benzyl oder zusammen $-(CH_2)_3-$, $-(CH_2)_4-$ oder $-(CH_2)_2-O-(CH_2)_2-$ darstellen und $Q_7$ Wasserstoff, geradkettiges oder verzweigtes $C_{1-8}$-Alkyl oder zusammen mit $Q_5$-$(CH_2)_q$- mit q = 3, 4 oder 5 bedeutet.

## Claims

1. A process for the preparation of stilbene-4,4'-dialdehyde by reacting ethylene with 4-bromobenzaldehyde in the presence of a base and of a palladium compound as catalyst, which process comprises performing the reaction under an ethylene partial pressure in the range from 0.01 to 1 bar.

2. A process according to claim 1, wherein the reaction is performed in the presence of a mixture of palladium acetate and tri-o-tolylphosphine as catalyst.

3. A process according to claim 2, wherein the reaction is performed under an ethylene partial pressure in the range from 0.1 to 1.0 bar.

4. A process according to claim 3, wherein the catalyst is used in an amount of 0.01 to 2 mol%, based on the 4-bromobenzaldehyde.

5. A process according to either of claims 1 or 4, wherein the reaction is performed at a temperature in the range from 30 to 200° C, preferably from 80 to 150° C, and in the presence of an organic solvent which is inert to the reactants.

6. A process according to claim 5, wherein the solvent is a cyclic or N,N-disubstituted amide.

7. A process according to either of claims 1 or 6, wherein the reaction is performed in the presence of a base.

8. A process according to claim 7, wherein the base is a compound of the formulae

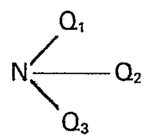 (I) or 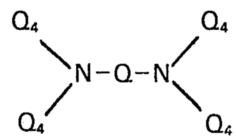 (II)

in which formulae

Q is straight chain or branched $C_2$-$C_6$ alkylene, $Q_1$ is $C_1$-$C_{12}$ alkyl, cyclopentyl, cyclohexyl or phenyl, or benzyl which can also be substituted, for example by a halogen atom such as chlorine or bromine or by a $C_1$-$C_4$ alkyl or $C_1$-$C_4$ alkoxy group, preferably a $C_1$-$C_2$ alkyl or $C_1$-$C_2$ alkoxy group, $Q_2$ and $Q_3$ are the same or different $C_1$-$C_{12}$ alkyl groups, and $Q_4$ is methyl or ethyl.

9. A process according to claim 8, wherein the base is tri-n-butylamine.

10. A process according to claim 6, wherein the solvent is a compound of the formula

$$Q_5Q_6NCOQ_7$$

wherein $Q_5$ and $Q_6$ independently of each other are straight chain or branched $C_1$-$C_8$ alkyl, $C_5$-$C_8$ cycloalkyl or benzyl, or together they are $-(CH_2)_3-$, $-(CH_2)_4-$ or $-(CH_2)_2-O-(CH_2)_2-$, and $Q_7$ is hydrogen, straight chain or branched $C_1$-$C_8$ alkyl, or together with $Q_5$ it is $-(CH_2)_q-$, wherein q is 3, 4 or 5.

## Revendications

1. Procédé de préparation du stilben-4,4'-dialdéhyde par réaction de l'éthylène avec le 4-bromobenzaldéhyde en présence d'une base et d'un composé du palladium comme catalyseur, caractérisé en ce qu'on effectue la réaction sous une pression partielle d'éthylène de 0,01 à 1 bar.

2. Procédé selon la revendication 1, caractérisé en ce qu'on effectue la réaction en présence d'un mélange d'acétate de palladium et de tri-o-tolylphosphine comme catalyseur.

3. Procédé selon la revendication 2, caractérisé en ce qu'on effectue la réaction sous une pression partielle d'éthylène comprise entre 0,1 et 1,0 bar.

4. Procédé selon la revendication 3, caractérisé en ce qu'on utilise le catalyseur dans une quantité de 0,01 à 2 mol%, par rapport au 4-bromobenzaldéhyde.

5. Procédé selon les revendications 1 et 4, caractérisé en ce qu'on effectue la réaction à une température comprise entre 30 et 200° C, de préférence entre 80 et 150° C, en présence d'un solvant organique inerte vis-à-vis des partenaires réactionnels.

6. Procédé selon la revendication 5, caractérisé en ce qu'on utilise comme solvant des amides cycliques ou N,N-disubstitués.

7. Procédé selon les revendications 1 et 6, caractérisé en ce qu'on effectue la réaction en présence d'une base.

8. Procédé selon la revendication 7, caractérisé en ce qu'on utilise comme base un composé répondant aux formules

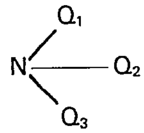 (I) ou 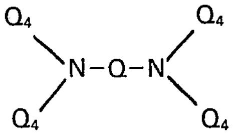 (II)

dans lesquelles Q désigne un groupe alkylène en $C_2$-$C_6$ linéaire ou ramifié, $Q_1$ désigne un groupe alkyle en $C_1$-$C_{12}$, un groupe cyclopentyle, cyclohexyle, phényle ou benzyle, qui peut aussi être substitué, par exemple par un atome d'halogène tel que le chlore ou le brome, ou un groupe alkyle ou alcoxy en $C_1$-$C_4$ et en particulier en $C_1$-$C_2$, et $Q_2$ et $Q_3$ désignent des groupes alkyle en $C_1$-$C_{12}$ identiques ou différents et $Q_4$ un groupe méthyle ou éthyle.

9. Procédé selon la revendication 8, caractérisé en ce qu'on utilise comme base de la tri-n-butylamine.

10. Procédé selon la revendication 6, caractérisé en ce qu'on utilise comme solvants des composés répondant à la formule

$$Q_5Q_6NCOQ_7$$

dans laquelle $Q_5$ et $Q_6$ désignent indépendamment l'un de l'autre un groupe alkyle en $C_1$-$C_8$ linéaire ou ramifié, un groupe cycloalkyle en $C_5$-$C_8$ ou un groupe benzyle, ou ensemble $-(CH_2)_3-$, $-(CH_2)_4-$ ou $-(CH_2)_2-O-(CH_2)_2-$ et $Q_7$ désigne l'hydrogène, un groupe alkyle en $C_1$-$C_8$ linéaire ou ramifié ou ensemble avec $Q_5$ $-(CH_2)_q-$, avec q = 3, 4 ou 5.